(19) Europäisches Patentamt
European Patent Office
Office européen des brevets

(11) EP 3 387 989 A1

(12) EUROPEAN PATENT APPLICATION

(43) Date of publication:
17.10.2018 Bulletin 2018/42

(51) Int Cl.:
A61B 5/024 (2006.01)          A61B 5/083 (2006.01)
A61B 5/11 (2006.01)           A61B 5/00 (2006.01)
G01N 33/00 (2006.01)          G06F 19/00 (2018.01)

(21) Application number: 17166438.6

(22) Date of filing: 13.04.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: Koninklijke Philips N.V.
5656 AE Eindhoven (NL)

(72) Inventor: TIEMANN, Christian Andreas
5656 AE Eindhoven (NL)

(74) Representative: de Haan, Poul Erik et al
Philips International B.V.
Philips Intellectual Property & Standards
High Tech Campus 5
5656 AE Eindhoven (NL)

(54) A METHOD AND APPARATUS FOR MONITORING A SUBJECT

(57) According to an aspect, there is provided a method of monitoring a subject to identify when the subject has eaten food, the method comprising obtaining measurements of the heart rate variability, HRV, of the subject over a first time interval; obtaining measurements of one or both of physical activity of the subject over the first time interval and measurements of a level of carbon dioxide in the environment of the subject over the first time interval; processing the HRV measurements and said one or both of the physical activity measurements and carbon dioxide, CO2, measurements to identify periods of time in which the subject has eaten food; and generating a signal indicative of the identified periods of time in which the subject has eaten food.

Figure 1

**Description**

TECHNICAL FIELD OF THE INVENTION

The invention relates to a method and apparatus for monitoring a subject, and in particular for monitoring a subject to identify when the subject has eaten food.

BACKGROUND OF THE INVENTION

The incidence of obesity and nutrition-related diseases is steadily increasing. A disturbed eating behavior, malnutrition and dehydration are also common problems for, amongst others, the elderly population, especially in people with dementia. Monitoring the food intake behavior of a subject/user is therefore relevant for health management programs and elderly care services. Eating behavior is an important parameter for a healthy lifestyle, and a disturbed eating behavior can have a severe impact on the health of the person. However, it is challenging to address this issue since it is difficult and cumbersome to assess eating behavior objectively.

Food intake is typically tracked using food intake questionnaires that require manual completion by the person. This procedure is very time consuming and not followed well by users. Users, for instance patients, may only be prompted once a day, and thus, many details of their eating behavior can be missed or forgotten. Furthermore, in cases of cognitive restrictions such as dementia, food intake logging has to be performed by a caregiver, the latter being also prone to missing a plurality of details of the user's eating behavior.

Hence, an improved method and apparatus for monitoring a subject to determine when they have eaten food would be useful within a number of settings, not limited to the ones set forth in the present document.

SUMMARY OF THE INVENTION

The invention is based on the finding that the heart rate variability (HRV) of a subject (also known as the RR interval) is affected by a subject eating food, and thus analysis of measurements of HRV could be used to identify when a subject has eaten food. However, HRV can vary for several reasons, and thus the reliability of identifying or detecting when the subject has eaten food can be improved by combining the analysis of the HRV measurements with measurements of one or more other parameters. In particular embodiments, the one or more other parameters can be selected from a measure of the physical activity of the subject and measurements of a level of carbon dioxide ($CO_2$) in the environment of the subject.

Thus, according to a first aspect, there is provided a method of monitoring a subject to identify when the subject has eaten food, the method comprising obtaining measurements of the heart rate variability, HRV, of the subject over a first time interval; obtaining measurements of one or both of the physical activity of the subject over the first time interval and measurements of a level of carbon dioxide in the environment of the subject over the first time interval; processing the HRV measurements and said one or both of the physical activity measurements and carbon dioxide, $CO_2$, measurements to identify periods of time in the first time interval in which the subject has eaten food; and generating a signal indicative of the identified periods of time in which the subject has eaten food. Thus the invention provides a method in which the subject can be monitored unobtrusively and automatically in order to determine if the subject has eaten food, thereby, amongst other things, increasing reliability and accuracy of the eating behavior associated information of the user.

In some embodiments, the step of processing comprises determining one or more HRV metrics from the HRV measurements, wherein the one or more HRV metrics comprises any one or more of (i) a standard deviation of heart beat intervals; (ii) a root mean square of successive differences between consecutive inter-beat intervals; (iii) a standard deviation of successive differences between consecutive inter-beat intervals; (iv) a number of pairs of successive inter-beat intervals that differ by more than y ms; (v) a proportion of the metric in (iv) divided by total number of inter-beat intervals; (vi) a number of pairs of successive inter-beat intervals that differ by more than z ms; (vii) a proportion of the metric in (vi) divided by total number of inter-beat intervals.

In some embodiments, the step of processing comprises determining one or more physical activity metrics from the measurements of physical activity, wherein the one or more physical activity metrics comprise one or more of a type of physical activity, a physical activity intensity and an energy expenditure.

In some embodiments, the step of processing comprises determining one or more CO2-based metrics from the measurements of $CO_2$, wherein the one or more CO2-based metrics comprise one or more of the total energy expenditure, TEE, the basal metabolic rate, BMR, and the thermic effect of food, TEF.

In some embodiments, the step of processing comprises processing the HRV measurements and said one or both of the physical activity measurements and CO2 measurements to determine a probability that the subject has eaten food in a particular time period in the first time interval; and determining whether the subject has eaten food in the particular time period based on the determined probability.

In some embodiments, the probability is determined using a logistic regression model, a neural network, or a deep learning technique.

In some embodiments, the step of processing comprises determining a probability that the subject has eaten food in a particular time period in the first time interval from the HRV measurements; scaling the probability based on the physical activity measurements and/or CO2 measurements; and comparing the scaled probability to a threshold value to determine if the subject has eaten food in the particular time period.

In some embodiments, the step of processing comprises determining a probability that the subject has eaten food in a particular time period in the first time interval from the HRV measurements and one or both of the physical activity measurements and/or CO2 measurements; and comparing the probability to a threshold value to determine if the subject has eaten food in the particular time period.

In some embodiments, the step of processing comprises determining a probability that the subject has eaten food in a particular time period in the first time interval from the HRV measurements; scaling a threshold value based on the physical activity measurements and/or CO2 measurements; and comparing the probability to the scaled threshold value to determine if the subject has eaten food in the particular time period.

According to a second aspect, there is provided a computer program product comprising a computer readable medium having computer readable code embodied therein, the computer readable code being configured such that, on execution by a suitable computer or processor, the computer or processor is caused to perform the method according to any of the embodiments described above. A computer program product may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

According to a third aspect, there is provided an apparatus for monitoring a subject to identify when the subject has eaten food, the apparatus comprising a processing unit configured to obtain measurements of the heart rate variability, HRV, of the subject over a first time interval; obtain measurements of one or both of physical activity of the subject over the first time interval and measurements of a level of carbon dioxide in the environment of the subject over the first time interval; and process the HRV measurements and said one or both of the physical activity measurements and carbon dioxide, CO2, measurements to identify periods of time in the first time interval in which the subject has eaten food. Thus the invention provides an apparatus by which the subject can be monitored unobtrusively and automatically in order to determine if the subject has eaten food, thereby, amongst other things, increasing reliability and accuracy of the eating behavior associated information of the user. Additionally or alternatively, an apparatus of the kind set forth herein requires limited or no active involvement from the user, which makes it easier to use, and less prone to unintentional forgetfulness.

In some embodiments, the processing unit is configured to determine one or more HRV metrics from the HRV measurements , wherein the one or more HRV metrics comprises any one or more of (i) a standard deviation of heart beat intervals; (ii) a root mean square of successive differences between consecutive inter-beat intervals; (iii) a standard deviation of successive differences between consecutive inter-beat intervals; (iv) a number of pairs of successive inter-beat intervals that differ by more than y ms; (v) a proportion of the metric in (iv) divided by total number of inter-beat intervals; (vi) a number of pairs of successive inter-beat intervals that differ by more than z ms; (vii) a proportion of the metric in (vi) divided by total number of inter-beat intervals.

In some embodiments, the processing unit is configured to process the physical activity measurements to determine one or more physical activity metrics from the measurements of physical activity, wherein the one or more physical activity metrics comprise one or more of a type of physical activity, a physical activity intensity and an energy expenditure.

In some embodiments, the processing unit is configured to process the CO2 measurements to determine one or more CO2-based metrics from the measurements of CO2, wherein the one or more CO2-based metrics comprise one or more of the total energy expenditure, TEE, the basal metabolic rate, BMR, and the thermic effect of food, TEF.

In some embodiments, the processing unit is configured to process the HRV measurements and said one or both of the physical activity measurements and CO2 measurements to determine a probability that the subject has eaten food in a particular time period in the first time interval; and to determine whether the subject has eaten food in the particular time period based on the determined probability.

In some embodiments, the probability is determined using a logistic regression model, a neural network, or a deep learning technique.

In some embodiments, the processing unit is configured to process the HRV measurements to determine a probability that the subject has eaten food in a particular time period in the first time interval from the HRV measurements; scale the probability based on the physical activity measurements and/or CO2 measurements; and compare the scaled probability to a threshold value to determine if the subject has eaten food in the particular time period.

In some embodiments, the processing unit is configured to determine a probability that the subject has eaten food in a particular time period in the first time interval from the HRV measurements and one or both of the physical activity measurements and/or CO2 measurements; and compare the probability to a threshold value to determine if the subject has eaten food in the particular time period.

In some embodiments, the processing unit is configured to determine a probability that the subject has eaten food in a particular time period in the first time interval from the HRV measurements; scale a threshold value based on the physical activity measurements and/or CO2 measurements; and compare the probability to the scaled threshold value to determine if the subject has eaten food in the particular time period.

According to a fourth aspect, there is provided a system for monitoring a subject to identify when the subject has eaten food, the system comprising a physiological characteristic sensor for measuring HRV of the subject over time; and an apparatus according to any of the above embodiments. Thus the invention provides a system by which the subject can be monitored unobtrusively and automatically in order to determine if the subject has eaten food, thereby, amongst other things, increasing reliability and accuracy of the eating behavior associated information of the user. Additionally or alternatively, an apparatus of the kind set forth herein requires limited or no active involvement from the user, which makes it easier to use, and less prone to unintentional forgetfulness.

In some embodiments, the system further comprises a physical activity sensor for measuring the physical activity of the subject over time; and/or a CO2 sensor for measuring a level of CO2 in the environment of the subject.

In some embodiments, the physiological characteristic sensor is the same sensor as the physical activity sensor.

BRIEF DESCRIPTION OF THE DRAWINGS

For a better understanding of the invention, and to show more clearly how it may be carried into effect, reference will now be made, by way of example only, to the accompanying drawings, in which:

Figure 1 is a block diagram of a system according to an embodiment;
Figure 2 is a flow chart illustrating a method according to the invention;
Figure 3 illustrates examples of particular metrics that can be used in various embodiments of the invention;
Figure 4 shows a relationship between a calculated thermic effect of food and consumption of food;
Figure 5 is a block diagram of an exemplary embodiment of the algorithm for monitoring a subject to identify when the subject has eaten food;
Figure 6 is a series of graphs illustrating the use of RR intervals to identify when a subject has eaten food; and
Figure 7 is a set of graphs illustrating the occurrence of physical activities and locations of those physical activities for a population of subjects.

DETAILED DESCRIPTION OF THE EMBODIMENTS

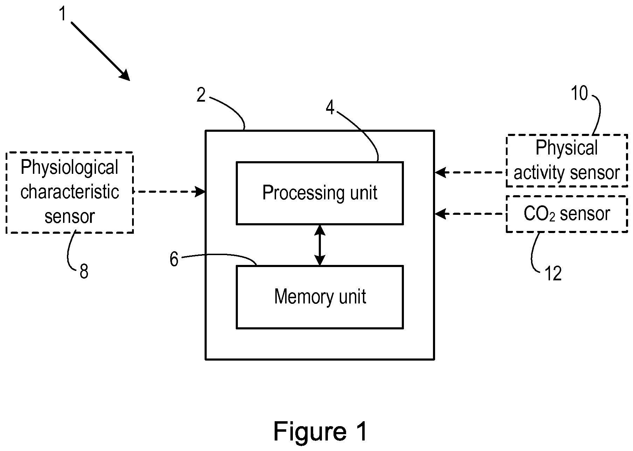

Figure 1 shows a system 1 for monitoring a subject to identify when the subject has eaten food according to an embodiment. Specifically, the system 1 is for identifying periods of time in which the subject has eaten food. The system 1 comprises an apparatus 2 that controls the system 1 and performs the operations and method according to the invention.

The apparatus 2 comprises a processing unit 4 that is for processing measurements to identify when the subject has eaten food and a memory unit 6 that can be used for storing measurements for processing and/or the results of the processing by the processing unit 4.

The processing unit 4 can be implemented in numerous ways, with software and/or hardware, to perform the various functions described below. The processing unit 4 may comprise one or more microprocessors or digital signal processors (DSPs) that may be programmed using software or computer program code to perform the required functions and/or to control components of the processing unit 4 to effect the required functions. The processing unit 4 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

The memory unit 6 may be a volatile or non-volatile computer memory such as RAM, PROM, EPROM, and EEPROM. The memory unit 6 can be used for storing program code that can be executed by a processor in the processing unit 4 to cause the apparatus 2 and/or system 1 to perform the method described herein. As noted above, the memory unit 6 can also be used to store signals or measurements for processing by the processing unit 4, and/or the results of that processing, such as an indication of whether the subject has eaten food at a particular time or times.

As noted above the invention is based on the finding that the heart rate variability (HRV) of a subject (also known as the RR interval) is affected by the subject eating food. Therefore the system 1 comprises a physiological characteristic sensor 8 that is for providing measurements of the HRV of the subject over time. As is known, the heart rate variability relates to the variation of the time interval between heart beats, which is referred to as variation in the beat-to-beat interval or inter-beat interval. The physiological characteristic sensor 8 may be a sensor that directly measures the inter-beat interval (HRV) of the subject or that measures a surrogate parameter (e.g. acceleration) from which the inter-beat interval (HRV) can be determined. For example the physiological characteristic sensor 8 can be a photoplethysmogram (PPG) sensor that measures the subject's pulse, an electrocardiogram (ECG) sensor (which can comprise a set of electrodes), a ballistocardiogram (BCG) sensor that can measure the subject's heart beat or blood flow, or a movement sensor that can measure movements caused by the beating of the heart and/or pulses of blood (e.g. an accelerometer, a strain gauge, etc.). The physiological characteristic sensor 8 maybe a sensor that is worn or carried by the subject (e.g. in the clothing, as part of an arm band, as part of a chest band, or a patch) or it can be a sensor that is located remotely from the subject (e.g. in the room that the subject is in). For example a camera or other imaging device can be used to remotely observe a subject and the video sequence processed to obtain a PPG measurement (this is known as remote PPG). The measurements of the HRV will be obtained for a time interval of interest, for example certain time intervals during a day (e.g. at scheduled meal times), an interval of a few hours or days, or continuously. It will be appreciated that the physiological characteristic sensor 8 can provide a time series of HRV measurements (e.g. a time series of HRV measurement samples). It will be appreciated that the sampling frequency should be high enough to enable beat-to-beat time to be measured. Sampling frequencies around 5 kHz or higher could be used. The measurements obtained by the physiological characteristic sensor 8 can be provided directly to the processing unit 4 for processing according to the invention to identify if the subject has eaten food (e.g. the processing can be in real-time or near real-time, i.e. as the measurements are obtained), or the measurements can be stored in the memory unit 6 for processing by the processing unit 4 at a later stage (for example at the end of each hour, day, or other desired period).

Also as noted above, to improve the reliability of identification of periods of time in which the subject has eaten food, the HRV of the subject can be analyzed in combination with measurements of one or more other parameters, in particular measurements of the physical activity of the subject and/or carbon dioxide ($CO_2$) in the environment of the subject. Therefore the system 1 can comprise one or more sensors for obtaining these measurements.

Thus, the system 1 can comprise a physical activity sensor 10 that provides measurements that are indicative of the physical activity of the subject or that can be processed to determine the physical activity of the subject. The measurements obtained by the physical activity sensor 10 can be provided directly to the processing unit 4 along with the other measurements for processing according to the invention in real-time or near real-time to identify if the subject has eaten food, or the measurements can be stored in the memory unit 6 for processing by the processing unit 4 at a later stage. The physical activity sensor 10 can be an accelerometer or other type of movement sensor, e.g. a gyroscope, a magnetometer or a position/location sensor, that is worn or carried by the user (e.g. in the clothing, as part of an arm band, as part of a chest band, or a patch), or it can be in the form of a camera or other imaging device that can be used to observe a subject, with the images or a video sequence of images being processed to identify the physical activity of the subject. The physical activity may relate to the type of physical activity that the subject is performing or undertaking and/or the level of physical activity that the subject is performing or undertaking. The types of physical activity that can be identified can be general activities such as walking, sitting down, running or lying down, being idle (sedentary), activities of a specific part of the subject's body, such as movements (or types of movements) of a hand or hands, an arm or arms (e.g. that might correspond to moving food to the mouth of the subject), the torso, the head and/or a leg or legs, or the posture of the subject, the subject's head, torso or other specific body part (e.g. arm). The level of physical activity can be a measure of the intensity of the physical activity (i.e. how active the subject is at that time), such as an activity count, approximate energy expenditure of the subject (which can be referred to as the Active Energy Expenditure, AEE), number of footsteps in a time period, etc. In some embodiments, the level/intensity of the physical activity can be determined from measurements of the heart rate (since heart rate is affected by the level of physical activity).

Those skilled in the art will be aware of various techniques that can be used to process measurements from an accelerometer, movement sensor, camera or other type of physical activity sensor to determine a measure of the physical activity of the subject, and thus further details are not provided herein. Those skilled in the art will also be aware of alternative measures of physical activity that can be obtained, and thus the above examples should not be considered exhaustive.

As with the measurements of the HRV, the measurements of the physical activity will be obtained for a time interval of interest (at least partially overlapping with the time interval for which measurements of HRV are obtained), for example certain time intervals during a day (e.g. at scheduled meal times), an interval of a few hours or days, or continuously. The physical activity sensor 10 can provide a time series of physical activity measurements (e.g. a time series of physical activity measurement samples). Any suitable sampling frequency could be used to generate the time series of physical activity measurements. It will be appreciated that the sampling frequency may be much lower than that used to measure the beat-to-beat interval. Alternatively, the measurements of the physical activity can relate to longer discrete time periods, for example the physical activity type/level for each minute, hour, etc.

In addition or alternatively to providing a physical activity sensor 10, the system 1 can comprise a carbon dioxide sensor 12 for providing measurements of the $CO_2$ in the environment around the subject. The measurements obtained by the $CO_2$ sensor 12 can be provided directly to the processing unit 4 along with the other measurements for processing according to the invention in real-time or near real-time to identify if the subject has eaten food, or the measurements can be stored in the memory unit 6 for processing by the processing unit 4 at a later stage. The $CO_2$ sensor 12 can be a nondispersive infrared (NDIR) sensor or a chemical $CO_2$ sensor which contains $CO_2$-sensitive polymer layers. The $CO_2$ sensor 12 can be worn or carried by the subject, e.g. on the clothing or near the mouth or nose of the subject (e.g. integrated into a pair of glasses), in order to obtain a direct measurement of the exhaled $CO_2$. Alternatively the $CO_2$ sensor 12 can be located in the environment in which the subject is located, for example in a particular room, such as a dining room or other room where food is typically consumed, and the amount of exhaled $CO_2$ in the air can be estimated. A suitable $CO_2$ sensor 12 maybe found in a home weather station or monitor.

As with the measurements of the HRV and physical activity, the measurements of the $CO_2$ will be obtained for a time interval of interest (at least partially overlapping with the time intervals for which measurements of HRV and physical activity are obtained), for example certain time intervals during a day (e.g. at scheduled meal times), an interval of a few hours or days, or continuously. The $CO_2$ sensor 12 can provide a time series of $CO_2$ measurements (e.g. a time series of $CO_2$ measurement samples). Any suitable sampling frequency can be used. In the case of an environment-located sensor, the sampling frequency could be as low as a measurement per minute. In the case of a subject-worn or carried $CO_2$ sensor, the sampling frequency is preferably high enough to measure $CO_2$ on a breath-by-breath basis. Alternatively, the measurements of the $CO_2$ can relate to longer discrete time periods, for example the $CO_2$ for each minute, hour, etc.

It will be appreciated from the above that the measurements of the HRV and measurements of the physical activity of the subject can be obtained using a single sensor, and thus it is not necessary for there to be multiple sensors for separately measuring the HRV and physical activity. For example, an accelerometer or other movement sensor carried or worn by the subject can be used to obtain measurements from which the HRV of the subject and the physical activity of the subject can be derived. Likewise, a camera or other imaging device can be used to remotely observe the subject and the images or video sequence processed to determine the measurements of the HRV and physical activity of the subject.

In some embodiments, in addition to measurements of the physical activity of the subject and/or measurements of the $CO_2$, measurements can be provided of the location of the subject over time can be obtained. The location can be measured, for example, in terms of which room in a building (e.g. house or care facility) the subject is located, or an absolute location, such as that provided by a satellite positioning system (e.g. GPS) or through triangulation measurements of radio frequency (RF) signals, such as cellular telecommunication system signals or Wi-Fi. In these embodiments, a dedicated location sensor may be provided in the system 1, or alternatively the apparatus 2 can obtained measurements or information on the location of the subject from another device, such as a smart phone, tablet, or smart watch carried or used by the subject.

In some embodiments, the apparatus 2 can be in the form of a general purpose electronic device, such as a smart phone, smart watch, tablet, laptop, desktop computer, or remote server. In other embodiments, the apparatus 2 can be dedicated to the task of monitoring the food intake of the subject, or dedicated to monitoring the subject in general. For example the apparatus 2 can be in the form of a hub or monitoring device that is present in the home of the subject.

In some embodiments, depending on the form factor of the apparatus 2, one or more of the physiological characteristic sensor 8, physical activity sensor 10 and $CO_2$ sensor 12 can be integral with the apparatus 2 (e.g. within the same housing or device). In the example where the apparatus 2 is in the form of a smart watch, the smart watch may comprise a sensor or sensors for measuring a physiological characteristic of the subject (e.g. heart rate, pulse) and physical activity. In embodiments where one or more of the physiological characteristic sensor 8, physical activity sensor 10 and $CO_2$ sensor 12 are separate from the apparatus 2, the apparatus 2 can comprise one or more interfaces or components that enable the apparatus 2 to receive the measurements from the sensors 8, 10, 12. For example the apparatus 2 can comprise an interface for establishing a wired signaling connection between the apparatus 2 and the sensors 8, 10, 12, or components (e.g. receiver or transceiver circuitry and associated antenna(s)) for enabling a wireless signaling connection (e.g. using a cellular standard, such as LTE, Wi-Fi, Bluetooth, etc.) between the apparatus 2 and the sensors 8, 10, 12. In this case, the sensors 8, 10, 12 may be provided with corresponding components (e.g. transmitter or transceiver circuitry and associated antenna(s)) for establishing a wireless signaling connection with the apparatus 2.

It will be appreciated by those skilled in the art that only the components of the system 1 and apparatus 2 required to explain the invention have been described above and illustrated in Figure 1, and practical implementations of the system 1 and apparatus 2 will include further components to those shown. For example the apparatus 2 and/or system 1 may comprise a power source, e.g. a battery, for powering the processing unit 4, or a plug or equivalent for connecting the apparatus 2 and/or system 1 to a mains power supply. As another example, the apparatus 2 and/or system 1 may comprise an output unit for outputting a signal indicative of periods of time in which a subject has eaten food. In some embodiments, the output unit can be in the form of a user interface component that can be used to output the results of the processing by the processing unit 4 to the subject or another interested party (e.g. a family member, a doctor or a caregiver). The user interface component may comprise a display screen, audio speaker, vibration element, one or more lights or light elements (e.g. light emitting diodes, LEDs), etc. The user interface component can be configured to receive a signal from the processing unit 4 indicating the results of the processing according to the invention. The user interface component can then use the signal to provide information to the user on the time periods in which the subject has eaten food in any suitable format. As another example, the output unit can be in the form of a signaling interface, for example a wireless transceiver or a wired connection port (such as an Ethernet port or USB port), that enables the processing unit 4 to communicate a signal indicative of the periods of time in which the subject has eaten food to another computer or storage device. The another computer or storage device may use the signal to log the information on the identified periods of time in a care plan module or a food intake log.

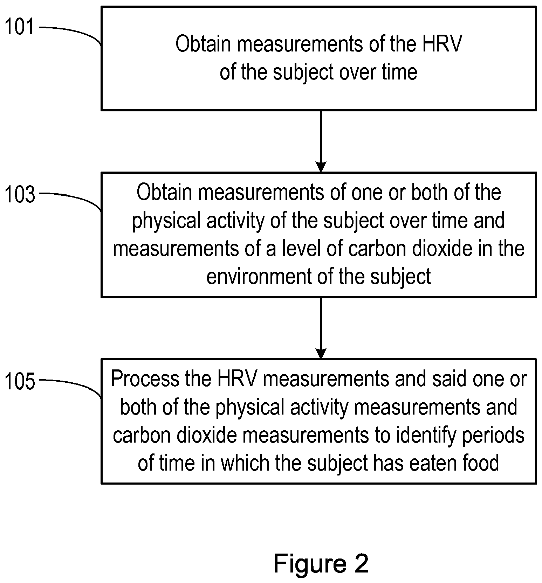

The flow chart in Figure 2 shows a method of monitoring a subject to identify when the subject has eaten food, and specifically identify periods of time in which the subject has eaten food according to an embodiment. The method can be performed by apparatus 2, and in particular by the processing unit 4.

In a first step, step 101, the processing unit 4 obtains measurements of the HRV of the subject over a time interval. Step 101 can comprise the processing unit 4 obtaining (e.g. retrieving) measurements from a memory unit 6, or receiving the measurements directly from the physiological characteristic sensor 8. In some embodiments, step 101 can comprise controlling the physiological characteristic sensor 8 to start making measurements of the HRV or surrogate HRV parameter.

Next, in step 103, the processing unit 4 obtains measurements of one or both of physical activity of the subject over a time interval and measurements of the $CO_2$ in the environment of the subject over a time interval. As with step 101, step 103 can comprise the processing unit 4 obtaining (e.g. retrieving) measurements from a memory unit 6, or receiving the measurements directly from the physical activity sensor 10 and/or $CO_2$ sensor 12. In some embodiments, step 103 can comprise controlling the physical activity sensor 10 and/or $CO_2$ sensor 12 to start making measurements of the relevant parameter relating to physical activity or $CO_2$ respectively.

It will be appreciated that the time interval(s) covered by the measurements obtained in step 101 overlap at least partly with the time interval(s) covered by the measurements obtained in step 103. The interval of time for which the measurements obtained in step 101 overlap with the measurement obtained in step 103 is referred to herein as the first time interval. Where steps 101 and 103 comprise the sensors 8, 10, 12 directly obtaining the measurements, it will be appreciated that steps 101 and 103 are performed at generally the same time.

In step 105, the HRV measurements obtained in step 101 and the one or both of the physical activity measurements and carbon dioxide measurements obtained in step 103 are processed to identify periods of time in the first time interval in which the subject has eaten food. Step 105 may provide an indication of a specific time period or time periods in which the subject has eaten, or may provide an indication that the subject has eaten food or not eaten food (as appropriate).

Next, a signal indicative of the periods of time identified in step 105 can be generated (e.g. by the processing unit 4). The signal can be used to log the information on the identified periods of time in a care plan module or a food intake log in the apparatus 2 or system 1. Alternatively, the generated signal can be provided by the processing unit 4 to a user interface component so that information on the identified periods of time can be presented to the subject or another user (e.g. using a display), or the signal can be provided to a signaling interface for communication of the signal to another computer or processor (for example that comprises a care plan module or food intake log).

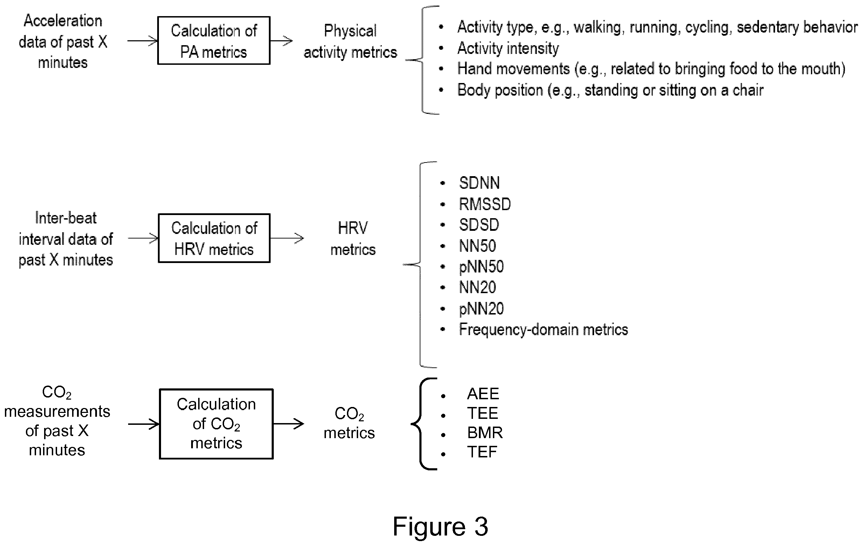

Step 105 can comprise identifying periods of time in the first time interval in which the subject has eaten food from one or more metrics that are derived from the measurements of HRV and physical activity and/or $CO_2$. Figure 3 illustrates various examples of HRV metrics, physical activity metrics and $CO_2$ metrics that can be derived by the processing unit 4 and used to identify when the subject has eaten food.

Thus, based on measurements of acceleration over an interval of time (e.g. X minutes), various physical activity (PA) metrics can be determined. Possible metrics include the activity type, e.g. walking, running, etc., the intensity of the activity (activity level), movements of a specific part of the body (e.g. hand) and/or body position.

Based on measurements of the inter-beat (RR) interval of the subject from the physiological characteristic sensor 8, various HRV metrics can be determined or calculated for a moving time window (e.g. X minutes in length). Examples of HRV metrics that can be determined or calculated include:

- SDNN: the standard deviation of RR intervals;
- RMSSD: root mean square of successive differences between consecutive RR intervals (i.e. the difference between successive data points, $(p(t) - p(t+1)$, $p(t+1) - p(t+2)$, etc.);
- SDSD: standard deviation of successive differences between consecutive RR intervals;
- NNy: the number of pairs of successive RR intervals that differ by more than y ms, where y can be, for example, in the range of 4-100 milliseconds, ms, e.g. 50 ms;

- pNNy: the proportion of NNy divided by total number of RR intervals;
- NNz: the number of pairs of successive RR intervals that differ by more than z ms where z can be, for example, in the range of 4-100 milliseconds, ms, e.g. 20 ms or 50 ms;
- pNNz: the proportion of NNz divided by total number of RR intervals.

In some embodiments, HRV metrics can be calculated based on an analysis of the inter-beat interval measurements in the frequency domain. Suitable frequency-domain HRV metrics can be obtained using a power spectral density (PSD) technique, a Fast Fourier Transform (FFT) or wavelet entropy analysis, for example in the frequency range of 0.0033 to 0.4 Hz.

Some of the above HRV metrics are described in the paper "Validation of the Use of Heart Rate Variability Measurements during Meal Intake in Humans" by Päßler et al., Computing in Cardiology 2013; 40:999-1002.

With regard to CO2, environmental CO2 can be affected by the energy expenditure of a person in the environment, and thus the measurements of environmental CO2 or the changes in environmental CO2 can be used to estimate the energy expenditure of the subject and metabolic rate. These metrics can be combined to estimate the thermic effect of food (TEF). In particular, food intake increases the energy expenditure due to the thermic effect of food. The metabolic rate is increased due to the digestion, absorption, and disposal of ingested macronutrients. The magnitude may be around 10% of the caloric intake, but it depends on the composition of the food. Carbohydrates can relate to 5 to 15% of the energy consumed, proteins to 20 to 35% of the energy consumed and lipids 5 to 15 % of the energy consumed.

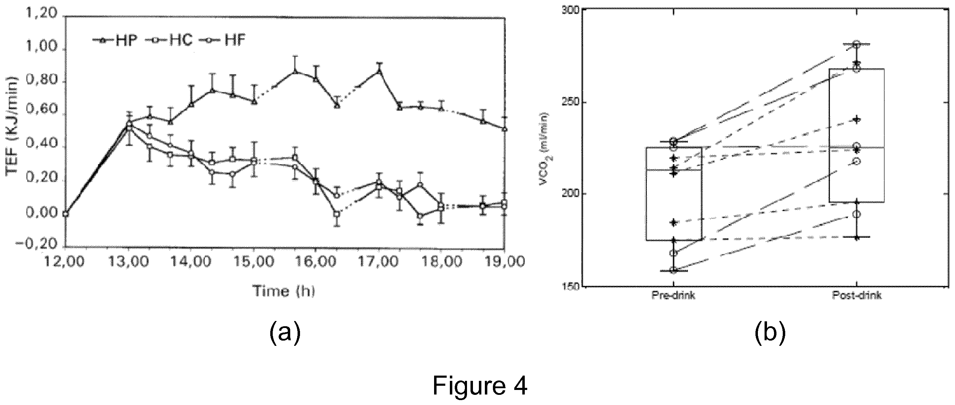

The graphs in Figure 4 show a relationship between a calculated thermic effect of food and consumption of food for different types of meal. In particular Figure 4(a) shows the TEF (measured in kilo Joules (kJ) per minute) over a period of several hours after the consumption of different types of meals (HP: high protein; HC: high carbohydrate; and HF: high fat). Figure 4(a) is found in "The influence of thermic effect of food on satiety" by Crovetti et al. Eur J Clin Nutr. 1998 Jul; 52(7):482-8. Figure 4(b) shows the increase in CO2 excretion (VCO2), measured in milliliters (ml) per minute) after intake of a high-caloric drink.

The thermic effect of food (TEF) can be calculated as follows:

$$TEF = TEE - AEE - BMR \qquad (1)$$

where TEE represents the total energy expenditure, AEE the active energy expenditure, and BMR the basal metabolic rate. TEE can be determined using a CO2 modelling approach, for example as described in WO 2017/006204. An increased energy expenditure is reflected in an increased excretion of CO2, resulting in an increased environmental CO2 level. AEE can be obtained from the measurements of the movements of the subject from the movement/physical activity sensor 10 as noted above. BMR can be calculated in several different ways. In a first way empirical formulas exist that can be used to estimate BMR based on age, gender, height, and weight. In a second way the CO2 modelling approach mentioned above could be used to estimate BMR during moments the subject is resting.

Step 105 may comprise calculating a probability from the obtained measurements that the subject has eaten food in a particular time period in the first time interval. In some embodiments, step 105 can comprise using a logistic regression model to determine the probability that the subject has eaten food in a particular time period in the first time interval.

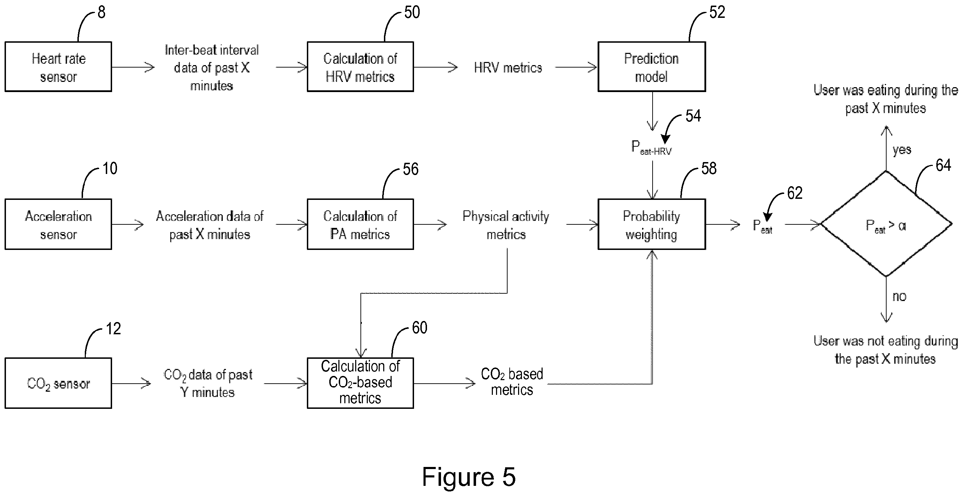

An exemplary algorithm for determining a probability that the subject has eaten food in a particular time period in the first time interval is described in more detail below with reference to Figure 5.

The core of the algorithm in Figure 5 is the use of HRV metrics and one or both of physical activity metrics and CO2-based metrics to determine if a subject is eating. The algorithm makes use of the fact that eating influences the autonomous nervous system (ANS) of the subject almost instantaneously, which is reflected in changes in heart rate variability. The HRV metrics are used as input in a logistic regression model. A number of HRV metrics such as SDNN, RMSSD, SDSD, etc. can be used.

Thus, in block 50 one or more HRV metrics are determined and input into a prediction model 52, such as a logistic regression model.

As an example, n HRV parameters denoted HRV1 to HRVn are input to the model 52. The probability that the subject has eaten food is denoted $P_{eat\text{-}HRV}$, and the logistic regression model can be defined as:

$$P_{eat\text{-}HRV} = e^{f(HRV)}/(1 + e^{f(HRV)}) \qquad (2)$$

$$= e^{(\beta 0 + \beta 1 HRV1 + ... + \beta n HRVn)} / (1 + e^{(\beta_0 + \beta_1 HRV1 + ... + \beta_n HRVn)}) \qquad (3)$$

The output of the model 52 is a probability value $P_{eat-HRV}$ 54 with a value between 0 and 1. The output behavior of the model is determined by parameters $\beta_0$ to $\beta_n$.

Values for these parameters can be defined to optimize (or at least provide an acceptably reliable outcome) the detection of 'eating' and 'not eating' events. Values for these parameters can be determined in a number of different ways. For example a training data set can be generated in which the heart rate variability of the subject of interest is monitored and the eating events are manually logged. The training data set can be evaluated to identify parameter values that provide a suitable reliability for eating detection. For example parameter values can be estimated using a least-squares technique that minimizes the difference between the manually logged data and the model output. Alternatively a training data set can be obtained for a population of people, and this data set evaluated to provide suitable parameter values. In some embodiments, a first estimate of the parameter values can be obtained using population data, and these first estimates refined using training data obtained for the subject of interest.

As noted above, in addition to food intake there are several processes that influence the autonomous nervous system and hence the HRV metrics, and so physical activity metrics and/or CO2-based metrics are used to improve the accuracy of the algorithm.

It is, for, instance not likely that a subject is eating while cycling or running, and so in this exemplary algorithm, $P_{eat-HRV}$ is adjusted based on the physical activity of the subject. Thus, in block 56 one or more metrics relating to physical activity are determined and these are input to a probability weighting block 58. The probability weighting block 58 also receives the $P_{eat-HRV}$ value 54 from the prediction model 52.

The physical activity metrics can be used as scaling factors, denoted $F_{activity}$, for the $P_{eat-HRV}$ value 54 (for example to increase or decrease the determined $P_{eat-HRV}$ value) in the probability weighting block 58. Values for the scaling factors for each type of physical activity metric (or each value of a physical activity metric, i.e. for each type of activity) can be determined. Values for these scaling factors could be determined from evaluation of a training data set for the subject and/or a population of subjects in the same way as for the parameter values $\beta_0$ to $\beta_n$ described above.

In some embodiments, conditional probabilities can be calculated, e.g., 'how often is it that a subject eats while performing a certain activity'. For activities such as cycling and running or some other sport, the probability is close to zero (since it would be physically difficult to eat a meal during those activities), and so $F_{activity}$ may reduce the probability to close to 0. For walking the probability is likely higher than for cycling or running, and so $F_{activity}$ maybe an intermediate value, but for most subjects, the subject is sitting relatively still during an eating event, and thus the value of the scaling factor $F_{activity}$ for that activity can provide a higher probability outcome (so $F_{activity}$ can be high or higher).

As well as the activity type, the movement intensity determined from the movement measurements can be useful for adjusting $P_{eat-HRV}$. The higher the physical activity intensity, the lower the probability that the subject is eating. Values for scaling factors for the activity level can be determined accordingly.

CO2-based metrics are calculated in block 60, particularly the thermic effect of food (TEF), and provided to the probability weighting block 58. The CO2-based metrics can be used as scaling factors, denoted $F_{TEF}$, for the $P_{eat-HRV}$ value 54 (for example to increase or decrease the determined $P_{eat-HRV}$ value) in the same way as described above for the physical activity metrics. Values for the scaling factors for each type of CO2-based metric (or each value of a CO2-based metric, i.e. for each of, e.g., TEF, TEE, BMR) can be determined. If the TEF value is high, $F_{TEF}$ will be high as well, and vice versa. Values for these scaling factors could be determined from evaluation of a training data set for the subject and/or a population of subjects in the same way as for the parameter values $\beta_0$ to $\beta_n$ described above.

It will be appreciated that there will be some delay between the time the subject starts to eat and the detection of an increased CO2 level due to TEF. This delay needs to be estimated based on the measured data, and corresponding eating event calculations should take this delay into account. The dynamics of TEF depend on the type of food eaten, as shown in Figure 4(a). The peak effect is typically between 1 and 2 hours after eating food, but the energy expenditure will typically start to increase much earlier, for example a few minutes after eating.

The output of the probability weighting block 58 is the probability Peat 62, and this can be determined, for example, according to:

$$P_{eat} = P_{eat-HRV} * F_{activity} * F_{TEF} \qquad (4)$$

This probability 62 can be compared to a threshold value in block 64 to determine if the subject was eating food in the time period for which the HRV measurements and the physical activity and/or CO2 measurements have been evaluated. This threshold is denoted $\alpha$. The threshold $\alpha$ can take any suitable or desired value, and in some embodiments the value of $\alpha$ is determined based on an evaluation or analysis of training data for the subject and/or a population of people. The value of the threshold $\alpha$ can also be set based on the desired specificity and/or sensitivity of the algorithm (e.g. based on the desired false-positive and false-negative rates). The algorithm can determine that the subject was eating food in a particular time period if the probability 62 exceeds the threshold $\alpha$, and determine that the subject was not eating food in the particular time period if the probability 62 is below the threshold. As an example, the threshold $\alpha$ can have the value of 0.5.

Further contextual data about the subject could be used in the algorithm to further reduce false-positives. For instance, the location of the subject can be useful, since, for example it is less likely that a subject is eating while driving a car. Another type of contextual data can be the time of day, for example it is more likely that a subject will eat during typical breakfast/lunch/dinner times, compared to midnight when the subject is expected to be sleeping. Yet another type of contextual data is the proximity of the time period of interest to another detected eating event. For example if the algorithm has detected that the subject has eaten a meal at a particular time, it may be unlikely that the subject has another meal within say, 2 or 3 hours of that meal (although this time frame may depend on the subject).

In an alternative embodiment of the algorithm, rather than combine the probability derived from the HRV, $P_{eat\text{-}HRV}$, with the scaling factors based on physical activity and/or CO2, the threshold value $\alpha$ could be adjusted or scaled using the scaling factors $F_{activity}$ and/or $F_{TEF}$. For example, if a scaling factor indicates that the subject is less likely to be eating, the threshold value $\alpha$ may be increased such that only high values of $P_{eat\text{-}HRV}$ result in the detection of an eating event.

In another alternative embodiment of the algorithm, rather than combine the probability derived from the HRV, $P_{eat\text{-}HRV}$, with the scaling factors based on physical activity and/or CO2, the physical activity metric(s) and/or the CO2 metric(s) can be directly input to the prediction model block 52 so that these metrics are used to form the eating probability. In this embodiment, the probability weighting block 58 may be omitted.

In yet further alternative embodiments, a combination of the above alternative embodiments can be used. Merely as an example, the physical activity metrics could be input directly to the prediction model block 52, and the CO2-based metrics could be used to adjust the threshold value $\alpha$, or vice versa. Other combinations of the embodiments will be evident to those skilled in the art.

In further alternative embodiments, the prediction model block 52 can implement an alternative type of model or data analysis technique to a logistic regression model. For example the prediction model block 52 can process the HRV metrics (and optionally the physical activity metrics and/or CO2-based metrics) using a neural network or deep learning techniques.

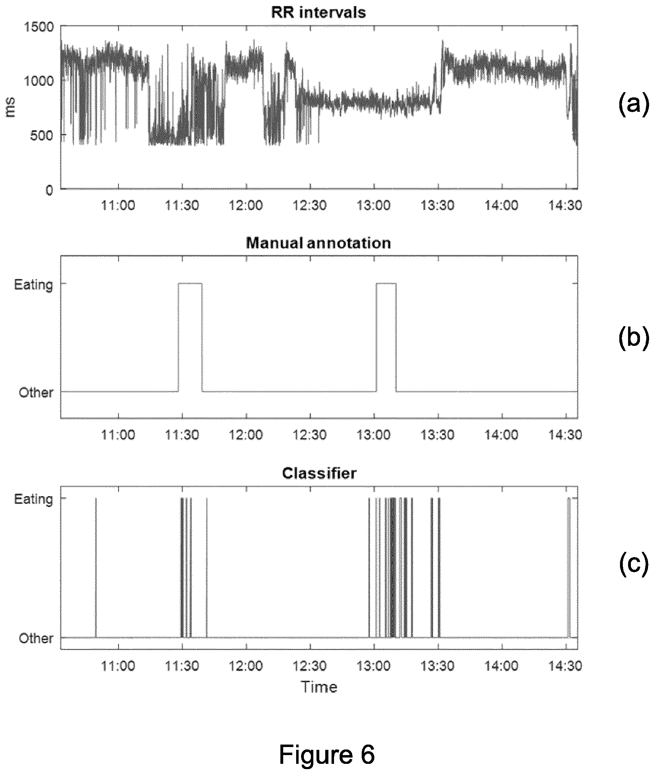

The graphs in Figure 6 illustrate the link between HRV and eating food based on measurements of beat-to beat intervals for a single subject. Figure 6(a) shows RR interval (beat-to-beat) data that has been obtained for a subject during a period of several hours. During that period the subject has eaten food (sandwiches) around 11:30 and around 13:00, and this has been manually noted by the subject, as shown in Figure 6(b).

Various HRV metrics were calculated as described above from the RR interval data, and these were input to a logistic regression model (classifier). The classifier output ($P_{eat\text{-}HRV}$ 54) is shown in Figure 6(c). This classifier was based on a subset selection technique in which iteratively significant predictors are added to the model (starting with just an offset). The set of possible predictors contained SDNN, RMSSD, SDSD, pNN50, pNN20, average RR (where all metrics are calculated on a rolling window of 1 minute). All these metrics also ended up in the final model. Peaks are visible in Figure 6(c) around the times that the subject manually noted that they were eating. Thus, it can be seen that there is a link between HRV metrics and eating food.

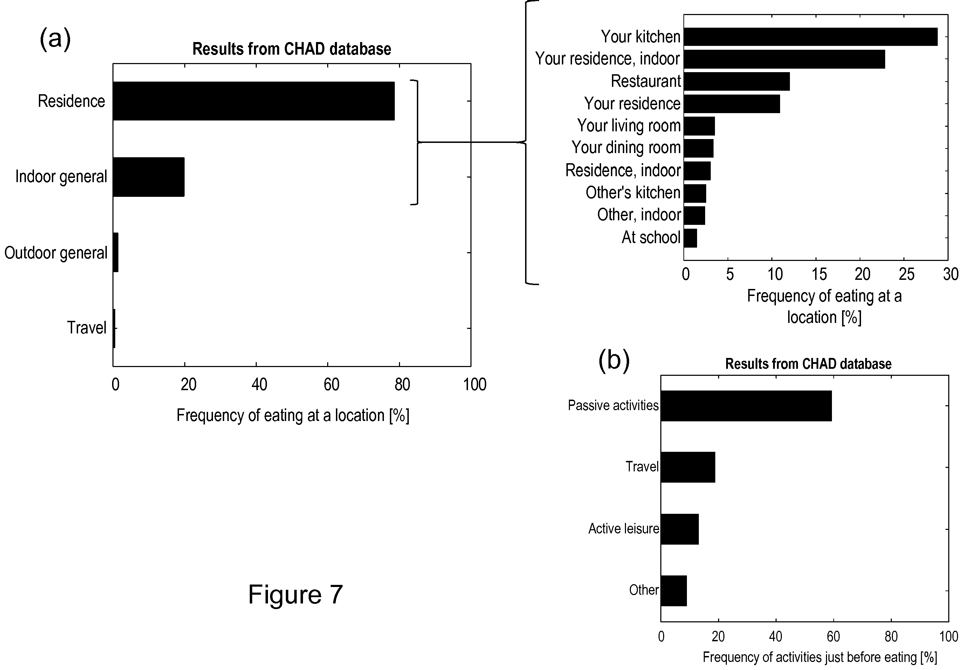

Figure 7 shows two graphs that illustrate the occurrence of physical activities and locations of those physical activities for a population of subjects. These graphs were derived from the Consolidated Human Activity Database (CHAD) from the US Environmental Protection Agency which comprises data sets relating to human behavior, with days broken down into hours and activity types. Figure 7(a) shows the frequency of eating at a particular location, namely their residence (home), indoors (generally), outdoors (generally) and while travelling, with the residence and indoor categories broken down into specific rooms, such as kitchen, dining room, a restaurant, etc. Figure 7(a) shows the types of activities that subject's perform just before an eating event, split into passive activities (including sitting down), travel, active activities and other. The data in Figures 7(a) and 7(b) suggests that subjects tend to eat indoors and perform passive activities around the eating event. Thus, scaling factors for these types of locations/passive behaviors can be high/higher, whereas for outdoor locations/active behaviors the scaling factors can be low/lower.

There is therefore provided an improved method and apparatus for monitoring a subject, particularly to identify a period or periods of time in which a subject has eaten food.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A method of monitoring a subject to identify when the subject has eaten food, the method comprising:

   obtaining measurements of the heart rate variability, HRV, of the subject over a first time interval;
   obtaining measurements of one or both of physical activity of the subject over the first time interval and measurements of a level of carbon dioxide in the environment of the subject over the first time interval;
   processing the HRV measurements and said one or both of the physical activity measurements and carbon dioxide, CO2, measurements to identify periods of time in the first time interval in which the subject has eaten food; and
   generating a signal indicative of the identified periods of time in which the subject has eaten food.

2. A method as claimed in claim 1, wherein the step of processing comprises determining one or more HRV metrics from the HRV measurements, wherein the one or more HRV metrics comprise any one or more of:

   (i) a standard deviation of heart beat intervals;
   (ii) a root mean square of successive differences between consecutive inter-beat intervals;
   (iii) a standard deviation of successive differences between consecutive inter-beat intervals;
   (iv) a number of pairs of successive inter-beat intervals that differ by more than y ms;
   (v) a proportion of the metric in (iv) divided by total number of inter-beat intervals;
   (vi) a number of pairs of successive inter-beat intervals that differ by more than z ms;
   (vii) a proportion of the metric in (vi) divided by total number of inter-beat intervals.

3. A method as claimed in claim 1 or 2, wherein the step of processing comprises determining one or more physical activity metrics from the measurements of physical activity, wherein the one or more physical activity metrics comprise one or more of a type of physical activity, a physical activity intensity and an energy expenditure.

4. A method as claimed in claim 1, 2 or 3, wherein the step of processing comprises determining one or more CO2-based metrics from the measurements of CO2, wherein the one or more CO2-based metrics comprise one or more of the total energy expenditure, TEE, the basal metabolic rate, BMR, and the thermic effect of food, TEF.

5. A method as claimed in any of claims 1-4, wherein the step of processing comprises:

   processing the HRV measurements and said one or both of the physical activity measurements and CO2 measurements to determine a probability that the subject has eaten food in a particular time period in the first time interval; and
   determining whether the subject has eaten food in the particular time period based on the determined probability.

6. A method as claimed in claim 5, wherein the probability is determined using a logistic regression model, a neural network, or a deep learning technique.

7. A computer program product comprising a computer readable medium having computer readable code embodied therein, the computer readable code being configured such that, on execution by a suitable computer or processor, the computer or processor is caused to perform the method of any of claims 1-6.

8. An apparatus for monitoring a subject to identify when the subject has eaten food, the apparatus comprising:

   a processing unit configured to:

   obtain measurements of the heart rate variability, HRV, of the subject over a first time interval;
   obtain measurements of one or both of physical activity of the subject over the first time interval and measurements of a level of carbon dioxide in the environment of the subject over the first time interval; and
   process the HRV measurements and said one or both of the physical activity measurements and carbon dioxide, CO2, measurements to identify periods of time in the first time interval in which the subject has eaten food;

   an output unit for outputting a signal indicative of the identified periods of time in which the subject has eaten food.

9. An apparatus as claimed in claim 8, wherein the processing unit is configured to determine one or more HRV metrics from the HRV measurements, wherein the one or more HRV metrics comprise any one or more of:

(i) a standard deviation of heart beat intervals;
(ii) a root mean square of successive differences between consecutive inter-beat intervals;
(iii) a standard deviation of successive differences between consecutive inter-beat intervals;
(iv) a number of pairs of successive inter-beat intervals that differ by more than y ms;
(v) a proportion of the metric in (iv) divided by total number of inter-beat intervals;
(vi) a number of pairs of successive inter-beat intervals that differ by more than z ms;
(vii) a proportion of the metric in (vi) divided by total number of inter-beat intervals.

10. An apparatus as claimed in claim 8 or 9, wherein the processing unit is configured to determine one or more physical activity metrics from the measurements of physical activity, wherein the one or more physical activity metrics comprise one or more of a type of physical activity, a physical activity intensity and an energy expenditure.

11. An apparatus as claimed in claim 8, 9 or 10, wherein the processing unit is configured to determine one or more $CO_2$-based metrics from the measurements of $CO_2$, wherein the one or more $CO_2$-based metrics comprise one or more of the total energy expenditure, TEE, the basal metabolic rate, BMR, and the thermic effect of food, TEF.

12. An apparatus as claimed in any of claims 8-11, wherein the processing unit is configured to:

process the HRV measurements and said one or both of the physical activity measurements and $CO_2$ measurements to determine a probability that the subject has eaten food in a particular time period in the first time interval; and
determine whether the subject has eaten food in the particular time period based on the determined probability.

13. A system for monitoring a subject to identify when the subject has eaten food, the system comprising:

a physiological characteristic sensor for measuring HRV of the subject over time; and
an apparatus as claimed in any of claims 8-12.

14. A system as claimed in claim 13, wherein the system further comprises:

a physical activity sensor for measuring the physical activity of the subject over time; and/or
a $CO_2$ sensor for measuring a level of $CO_2$ in the environment of the subject.

15. A system as claimed in claim 14, wherein the physiological characteristic sensor is the same sensor as the physical activity sensor.

1

2      4                                    10

| Physiological characteristic sensor |     | Processing unit |     | Physical activity sensor |
|                                     |     |                 |     | CO₂ sensor               |

8                    6                              12

Memory unit

Figure 1

101 — Obtain measurements of the HRV
of the subject over time

103 — Obtain measurements of one or both of the
physical activity of the subject over time and
measurements of a level of carbon dioxide in the
environment of the subject

105 — Process the HRV measurements and said one or
both of the physical activity measurements and
carbon dioxide measurements to identify periods
of time in which the subject has eaten food

## Figure 2

Acceleration data of past X minutes → Calculation of PA metrics → Physical activity metrics

- Activity type, e.g., walking, running, cycling, sedentary behavior
- Activity intensity
- Hand movements (e.g., related to bringing food to the mouth)
- Body position (e.g., standing or sitting on a chair

Inter-beat interval data of past X minutes → Calculation of HRV metrics → HRV metrics

- SDNN
- RMSSD
- SDSD
- NN50
- pNN50
- NN20
- pNN20
- Frequency-domain metrics

$CO_2$ measurements of past X minutes → Calculation of $CO_2$ metrics → $CO_2$ metrics

- AEE
- TEE
- BMR
- TEF

Figure 3

(a)

(b)

Figure 4

EP 3 387 989 A1

EP 3 387 989 A1

8

Heart rate sensor → Inter-beat interval data of past X minutes →

50

Calculation of HRV metrics → HRV metrics →

52

Prediction model

54

$P_{eat-HRV}$

10

Acceleration sensor → Acceleration data of past X minutes →

56

Calculation of PA metrics → Physical activity metrics →

58

Probability weighting → $P_{eat}$

62

64

$P_{eat} > \alpha$

yes → User was eating during the past X minutes

no → User was not eating during the past X minutes

12

$CO_2$ sensor → $CO_2$ data of past Y minutes →

60

Calculation of $CO_2$-based metrics → $CO_2$ based metrics

Figure 5

**RR intervals** (a)

**Manual annotation** (b)

**Classifier** (c)

Figure 6

Figure 7

EP 3 387 989 A1

Europäisches
Patentamt

European
Patent Office

Office européen
des brevets

## EUROPEAN SEARCH REPORT

**Application Number**

EP 17 16 6438

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/038585 A1 (BLACKTREE FITNESS TECHNOLOGIES INC [CA]) 17 March 2016 (2016-03-17) * abstract * * page 23, line 11 - page 27, line 8 * * page 41, lines 10-20 * * figures 1-4 * ----- | 1-15 | INV. A61B5/024 A61B5/083 A61B5/11 A61B5/00 ADD. G01N33/00 G06F19/00 |
| X | US 2009/105560 A1 (SOLOMON DAVID [IL]) 23 April 2009 (2009-04-23) * paragraphs [0014] - [0018], [0034], [0036], [0046], [0048], [0051] - [0054] * ----- | 1-3, 7-10, 13-15 | |
| A | US 2002/109600 A1 (MAULT JAMES R [US] ET AL) 15 August 2002 (2002-08-15) * the whole document * ----- | 1-15 | |

**TECHNICAL FIELDS SEARCHED (IPC)**

A61B
G01N
G06F

The present search report has been drawn up for all claims

| Place of search | Date of completion of the search | Examiner |
|---|---|---|
| The Hague | 28 September 2017 | Kowalczyk, Szczepan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 16 6438

This annex lists the patent family members relating to the patent documents cited in the above-mentioned European search report.
The members are as contained in the European Patent Office EDP file on
The European Patent Office is in no way liable for these particulars which are merely given for the purpose of information.

28-09-2017

| Patent document cited in search report | Publication date | Patent family member(s) | Publication date |
|---|---|---|---|
| WO 2016038585 A1 | 17-03-2016 | US 2017249445 A1<br>WO 2016038585 A1 | 31-08-2017<br>17-03-2016 |
| US 2009105560 A1 | 23-04-2009 | NONE | |
| US 2002109600 A1 | 15-08-2002 | AU 4337002 A<br>CA 2426681 A1<br>EP 1333755 A2<br>JP 2004515291 A<br>US 2002109600 A1<br>WO 0247465 A2 | 24-06-2002<br>20-06-2002<br>13-08-2003<br>27-05-2004<br>15-08-2002<br>20-06-2002 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017006204 A

**Non-patent literature cited in the description**

- **PÄßLER et al.** Validation of the Use of Heart Rate Variability Measurements during Meal Intake in Humans. *Computing in Cardiology,* 2013, vol. 40, 999-1002

- The influence of thermic effect of food on satiety" by Crovetti. *Eur J Clin Nutr.,* July 1998, vol. 52 (7), 482-8